# EUROPEAN PATENT SPECIFICATION

(11) **EP 2 625 286 B1**
(45) Date of publication and mention of the grant of the patent: **15.04.2020**
(21) Application number: 11763931.0
(22) Date of filing: 30.09.2011
(51) Int. Cl.: C12Q 1/6851

(54) **METHOD FOR CELL LYSIS IN A PCR REACTION BUFFER**
VERFAHREN FÜR ZELLLYSE IN EINEM PCR-REAKTIONSPUFFER
MÉTHODE DE LYSE CELLULAIRE DANS UN TAMPON DE RÉACTION DE PCR

(30) Priority: 04.10.2010 EP 10186416
(43) Date of publication of application: 14.08.2013
(73) Proprietor: Roche Diagnostics GmbH, 68305 Mannheim (DE); F. Hoffmann-La Roche AG, 4070 Basel (CH)
(72) Inventor: HOFFMANN, Ingrid, 83670 Bad Heilbrunn (DE)
(74) Representative: Merkel, Patrick
(86) International application number: PCT/EP2011/067069
(87) International publication number: WO 2012/045670

(56) References cited:
- WO-A1-2009/021215
- WO-A1-2010/040831
- YONGZHONG LI ET AL: "An improved one-tube RT-PCR protocol for analyzing single-cell gene expression in individual mammalian cells", ANALYTICAL AND BIOANALYTICAL CHEMISTRY, SPRINGER, BERLIN, DE, vol. 397, no. 5, 20 May 2010 (2010-05-20), pages 1853-1859, XP019839291, ISSN: 1618-2650
- CHRISTOPHER MARLOWE A CAIPANG ET AL: "Rapid diagnosis of vibriosis and white spot syndrome (WSS) in the culture of shrimp, Penaeus monodon in Philippines", VETERINARY RESEARCH COMMUNICATIONS ; AN INTERNATIONAL JOURNAL PUBLISHING TOPICAL REVIEWS AND RESEARCH ARTICLES ON ALL ASPECTS OF THE VETERINARY SCIENCES, KLUWER ACADEMIC PUBLISHERS, DO, vol. 34, no. 7, 28 July 2010 (2010-07-28), pages 597-605, XP019832155, ISSN: 1573-7446
- STAHLBERG A ET AL: "Single-cell gene expression profiling using reverse transcription quantitative real-time PCR", METHODS : A COMPANION TO METHODS IN ENZYMOLOGY, ACADEMIC PRESS INC., NEW YORK, NY, US, vol. 50, no. 4, 1 April 2010 (2010-04-01), pages 282-288, XP026941721, ISSN: 1046-2023, DOI: DOI:10.1016/J.YMETH.2010.01.002 [retrieved on 2010-03-06]
- PEIXOTO ANTÓNIO ET AL: "Quantification of multiple gene expression in individual cells", GENOME RESEARCH, COLD SPRING HARBOR LABORATORY PRESS, WOODBURY, NY, US, vol. 14, no. 10A, 1 October 2004 (2004-10-01), pages 1938-1947, XP002545890, ISSN: 1088-9051, DOI: DOI:10.1101/GR.2890204
- BENGTSSON M ET AL: "Gene expression profiling in single cells from the pancreatic islets of Langerhans reveals lognormal distribution of mRNA levels", GENOME RESEARCH, COLD SPRING HARBOR LABORATORY PRESS, WOODBURY, NY, US, vol. 15, no. 10, 1 October 2005 (2005-10-01), pages 1388-1392, XP002438658, ISSN: 1088-9051, DOI: DOI:10.1101/GR.3820805
- BLIND ET AL: "Differential recruitment of glucocorticoid receptor phospho-isoforms to glucocorticoid-induced genes", JOURNAL OF STEROID BIOCHEMISTRY AND MOLECULAR BIOLOGY, ELSEVIER SCIENCE LTD., OXFORD, GB, vol. 109, no. 1-2, 19 January 2008 (2008-01-19), pages 150-157, XP022534002, ISSN: 0960-0760, DOI: 10.1016/J.JSBMB.2008.01.002
- SASTRE J ET AL: "Age-associated oxidative damage leads to absence of gamma-cystathionase in over 50% of rat lenses: Relevance in cataractogenesis", FREE RADICAL BIOLOGY AND MEDICINE, ELSEVIER SCIENCE, US, vol. 38, no. 5, 1 March 2005 (2005-03-01), pages 575-582, XP025352055, ISSN: 0891-5849 [retrieved on 2005-03-01]
- AGÜERO MONTSERRAT ET AL: "A highly sensitive and specific gel-based multiplex RT-PCR assay for the simultaneous and differential diagnosis of African swine fever and Classical swine fever in clinical samples.", VETERINARY RESEARCH 2004 SEP-OCT LNKD- PUBMED:15369658, vol. 35, no. 5, September 2004 (2004-09), pages 551-563, XP002663152, ISSN: 0928-4249
- GRIMMLER C ET AL: "Transcriptional analysis of catabolite repression in Clostridium acetobutylicum growing on mixtures of d-glucose and d-xylose", JOURNAL OF BIOTECHNOLOGY, ELSEVIER SCIENCE PUBLISHERS, AMSTERDAM, NL, vol. 150, no. 3, 29 September 2010 (2010-09-29), pages 315-323, XP027483999, ISSN: 0168-1656, DOI: 10.1016/J.JBIOTEC.2010.09.938 [retrieved on 2010-09-29]
- CHEUNG T K W ET AL: "Evaluation of novel H1N1-specific primer-probe sets using commercial RT-PCR mixtures and a premixed reaction stored in a lyophilized format", JOURNAL OF VIROLOGICAL METHODS, ELSEVIER BV, NL, vol. 165, no. 2, 1 May 2010 (2010-05-01), pages 302-304, XP026993889, ISSN: 0166-0934 [retrieved on 2010-02-06]
- LORAM L C ET AL: "Intrathecal injection of an alpha seven nicotinic acetylcholine receptor agonist attenuates gp120-induced mechanical allodynia and spinal pro-inflammatory cytokine profiles in rats", BRAIN, BEHAVIOR AND IMMUNITY, ACADEMIC PRESS, SAN DIEGO, CA, US, vol. 24, no. 6, 1 August 2010 (2010-08-01) , pages 959-967, XP027118312, ISSN: 0889-1591 [retrieved on 2010-07-02]
- GOMEZ D I ET AL: "Systematic interpretation of molecular beacon polymerase chain reaction for identifying rpoB mutations in Mycobacterium tuberculosis isolates with mixed resistant and susceptible bacteria", DIAGNOSTIC MICROBIOLOGY AND INFECTIOUS DISEASES, ELSEVIER SCIENCE PUBLISHING CO., AMSTERDAM, NL, vol. 67, no. 1, 1 May 2010 (2010-05-01), pages 37-46, XP026995886, ISSN: 0732-8893 [retrieved on 2010-04-09]
- STÅHLBERG ANDERS ET AL: "Quantitative transcription factor analysis of undifferentiated single human embryonic stem cells.", CLINICAL CHEMISTRY DEC 2009 LNKD- PUBMED:19815608, vol. 55, no. 12, December 2009 (2009-12), pages 2162-2170, XP55012180, ISSN: 1530-8561
- YUAN GONG ET AL: "Massively parallel detection of gene expression in single cells using subnanolitre wells", LAB ON A CHIP, vol. 10, no. 18, 1 January 2010 (2010-01-01), page 2334, XP55012182, ISSN: 1473-0197, DOI: 10.1039/c004847j
- TETSUTARO HAYASHI ET AL: "Single-cell gene profiling of planarian stem cells using fluorescent activated cell sorting and its "index sorting" function for stem cell research", DEVELOPMENT, GROWTH & DIFFERENTIATION, vol. 52, no. 1, 1 January 2010 (2010-01-01), pages 131-144, XP55012184, ISSN: 0012-1592, DOI: 10.1111/j.1440-169X.2009.01157.x
- WARREN LUIGI ET AL: "Transcription factor profiling in individual hematopoietic progenitors by digital RT-PCR", PROCEEDINGS OF THE NATIONAL ACADEMY OF SCIENCES OF USA, NATIONAL ACADEMY OF SCIENCE, WASHINGTON, DC; US, vol. 103, no. 47, 1 November 2006 (2006-11-01), pages 17807-17812, XP002479982, ISSN: 0027-8424, DOI: 10.1073/PNAS.0608512103

## Description

### Field of the Invention

The invention relates to the field of DNA analysis by means of PCR. More specifically, the present invention provides a new method in order to perform analysis of DNA starting from a material of only a few cells, and subsequent direct analysis of said sample DNA by means of real time PCR.

### Background of the Invention

In the past decades, PCR has become the "working horse" for the analysis of DNA since it enables exponential amplification of nucleic acids. In particular real time PCR (also termed qPCR) has become a powerful tool since it enables simultanous analysis of the amplified nucleic acid during the amplification, or without intermediate opening, directly subsequent to the amplification reaction by means of melting curve analysis.

Moreover, automatization of PCR has made significant progress, since qPCR systems are now available which enable performance of 96, 384 or 1536 reactions in parallel in microtiter plate formates. Systems have also become more user friendly. For example, microtiter plates are commercially available, wherein each reaction vessel already comprises the compounds necessary to perform PCR amplification or PCR amplification and detection in a freeze dried form, and the customer only has to add the sample comprising the DNA to be analyzed, prior to the actual reaction itself. An alternative system is provided by Advalytics (AG 480F) which enables PCR amplifcation and detection on glass slides.

Nevertheless, it is still a challenge to further improve the work flow for PCR analysis, in particular, if analysis can only be performed on DNA originating from only a small number of cells. In traditional real time PCR, DNA or RNA is first isolated from cells in a time consuming procedure that can lead to a loss of material. Afer harvesting, the cells are lysed and the DNA is at least partially purified from the lysate, because otherwise, PCR amplification might be inhibited at least quantitatively. Approaches described in the art include the disclosure of Marlowe et al. (2010, Vet Res Comm, vol. 34, no. 7) relating to direct colony PCR on 30 bacterial isolates and duplex PCR on 10 total DNA samples. Stahlberg et al. (2010, Methods, vol. 50, no. 4) discloses single cell gene expression profiling using RT-qPCR, and further single cell sorting using flowcytometry, thereby resulting in samples comprising a liquid with a first volume and one living cell. WO2009/021215 A1 describes a kit comprising a plurality of reaction vessels (nanowells) designed to fit in a thermocycler instrument, said vessels being physically connected in the form of a plate and pre-loaded with the necessary PCR reagents and fluorescent detectors or markers. Peixoto et al. (2005, Genome Research, vol. 13, no. 10A) describes a kit comprising a plurality of reaction vessels (tubes for real-time qPCR) and SYBRGreen PCR Master mix comprising a double-stranded DNA-binding fluorescentcompound (SYBR Green), a thermostable DNA-dependent DNA molecule(AmpliTaq), dNTPs and PCR buffer, and a primer mix including at least one pair of primers. Hayashi et al. (2010, Development, Growth and Differentiation, vol. 52, no. 1) discloses a method for single-cell gene profiling of planarian stem cells usingfluorescent activated cell sorting (FACS), while using an "index sorting" function. Luigi et al. (2006, PNAS, vol. 103, no. 47) describes a further method using cell sorting for collecting samples of one or more living cells. WO 2010/040831 A1 discloses a method for amplification of a single-copy DNA target (human p53) from cells after seeding them in wells of a microwell plate.

In this context, the technical problem underlying the present invention was to provide an improved and automatable high throughput method which allows for a further simplified DNA analysis protocol.

### Summary of the Invention

The present invention provides a method for amplification of a single copy DNA target nucleic acid from one single living eucaryotic cell of human, animal or plant origin, comprising the steps of a) transfering a liquid sample with a first volume comprising the single living eucaryotic cell into a a well of a microtiter plate, b) adding to said well a PCR reaction buffer with a second volume, whereas said second volume is at least 2x as large as said first volume and the final volume does not exceed 25 µl, said buffer comprising at least one pair of amplification primers, a thermostable DNA polymerase and dNTPs, c) incubating said well for at least 30 seconds to not more than 15 minutes at at least 90°C, thereby lysing the living cell in the PCR reaction buffer without any prior addition of a specific lysis step reagent and activating the activity of said thermostable DNA polymerase, d) amplifying said target by means of a polymerase chain reaction with a thermostable DNA dependent DNA polymerase without performance of an intermediate purification step, wherein steps a) to d) are performed within the same reaction vessel.

Said PCR reaction buffer of step b) may in addition comprise a labeled hybridization probe or a double strand DNA binding fluorescent compound.

In one embodiment, the thermostable DNA Polymerase carries a chemical modification which is removed from said polymerase during step c).

In one embodiment of the inventive method, said liquid comprising the single living cell has been gained by a cell sorting method prior to step a).

Preferably, the ratio of the number of said living cells of step a) versus the liquid volume in which the polymerase chain reaction of step d) is perfomed is not greater than 2 cells/µl.

Further disclosed herein are specific kits useful for performance of the inventive method disclosed above.

### Detailed Description of the Invention

Generally speaking, the present invention provides a method which enables lysis of a cell sample in a liquid environment that later on is used directly for nucleic analysis by means of applying the Polymerase Chain reaction without any intermediate purification step or complex liquid handling procedures. More precisely, the present invention provides a method for amplification of a single copy DNA target nucleic acid from one single living eucaryotic cell of human, animal or plant origin, comprising the steps of a) transfering a liquid sample with a first volume comprising the single living eucaryotic cell into a a well of a microtiter plate, b) adding to said well a PCR reaction buffer with a second volume, whereas said second volume is at least 2x as large as said first volume and the final volume does not exceed 25 µl, said buffer comprising at least one pair of amplification primers, a thermostable DNA polymerase and dNTPs, c) incubating said well for at least 30 seconds to not more than 15 minutes at at least 90°C, thereby lysing the living cell in the PCR reaction buffer without any prior addition of a specific lysis step reagent and activating the activity of said thermostable DNA polymerase, d) amplifying said target by means of a polymerase chain reaction with a thermostable DNA dependent DNA polymerase without performance of an intermediate purification step, wherein steps a) to d) are performed within the same reaction vessel.

Preferably, step c) lasts at least minute.

The target nucleic acid is DNA, i.e. a specific part of the genomic DNA. The polymerase chain reaction will be carried out with a DNA dependent DNA Polymerase such as Taq DNA polymerase or the like. The specific part of the DNA may be amplified in order to identify any type of genomic variations such as single nucleotide polymorphisms and the like.

As it will be demonstrated by the examples, the present invention provides a method which is surprisingly applicable to perform a PCR analysis starting from only one single living cell as original sample material. Moreover it is possible to reproducibly amplify and analyze target DNA from a single copy gene from only a few cells or even one single cell.

### Step a)

The one or more living cells according to step a) are preferably eucaryotic cells of human, animal or plant origin. The cells may be derived from cell lines or biopsies.

The liquid containing the cells may be any liquid, buffer or medium in which said cells survive for at least a certain period of time, which is preferably longer than 30 minutes. Such a liquid, for example may be a medium in which cells living and growing in suspension have been successfully cultivated. In case of adherent cells, the liquid may be a buffer, in which the cells have been detached from the solid support. Preferably, however such a buffer is free of any proteases which might have an inhibitory effect on the subsequent polymerase mediated PCR amplification reaction. Such an effect, if observed, can be avoided by subjecting the cells to an additional washing step prior to deposition into the vessel.

The transfer of the liquid into the vessel may be achieved either manually by means of first preparing an appropriate dilution series of a cell sample and then pipeting an equivalent of only one or several cells into said vessel. Preferably, the transfer is achieved using an appropriate automated pipetting station or, mostly preferred, a cell sorter. Such cell sorting machines are well known in the art and commercially available from a number of different manufacturers. Due to the underlying technology of cell sorters, it will happen from time to time that large particles of cell debris are mistakenly recognized by the cell sorters as cells. Due to this effect, in case of single cell analysis some samples may give no results during the subsequent PCR reaction.

Preferably, the number of cells transferred to the reaction vessel should be limited since without any purification step, the presence of cellular debris in higher concentrations may inhibit the subsequent PCR amplifciation reaction. Advantageously, the number of cells transferred to the reaction vessel is adjusted so that step d) as disclosed below is performed in such a way that the sample does not exceed a ratio of 2 cell equivalents/µl.

Also preferably, the number of cells transferred to the reaction vessel should not be too low, because otherwise it may become difficult to perfom a PCR reaction for the amplification of single copy genes. Thus, the number of cells transferred to the reaction vessel is adjusted so that step d) as disclosed below is performed in such a way that the sample comprises a ratio of at least 1 cell equivalents/ 25 µl.

The volume of the liquid is sufficiently small such that addition of the PCR reaction buffer in step b) and - if required - addition of further compounds required for the subsequent PCR reaction finally result in a final volume which is still reasonable small for performance of said PCR. Said volume should never exceed 100 and by no means 200µl. Advantageously, said volume is not more than 50µl.

Highly preferred are volumes of 20 µl or less, and even 10 µl. In case of automated deposition of the liquid containing the one or more cells can be very low in sub-µl range. In particular, if a cell sorter is used, a volume containing only one cell equivlent is about less than 100 nl. Even if in the latter case the sample is dried out, it has been proven by the inventor that the new method is still effective. In the particular embodiment of single cell analysis, the final volume preferably does not exceed 25µl. This still allows for efficientz single copy gene amplification.

The reaction vessel may be any type of reaction vessel, in which a PCR reaction can be performed. Therefore, the only essential limitation is that the vessel has sufficient heat resistance, since during the PCR thermocycling protocol it will become repeatedly exposed to high temperatures of 90°C or above.

According to the present invention, the reaction vessel is a well of a microtiter plate which is suited or designed to be placed into a thermocycler instrument. This allows to execute the method of the present invention on a number of samples in a highly parallel manner. Microtiter plates comprising 24, 96, 384, and 1536 wells are known in the art and commercially available from a multitude of different suppliers. Microtiter plates available in the art allow for reaction volumes of at least 2 µl. Such microtiter plates can be subjected to high temperature of at least 90°C by means of placing them on an appropriate heating block, or, alternatively, directly into a PCR Thermocycler instrument which is designed to incorporate microtiter plates.

In some cases, the reaction vessel may be a single reaction tube or a reaction tube which is a part of a stripe of reaction tubes that are connected to each other so that they can jointly be placed into the heating block of a thermocycler instrument. In other cases, the reaction vessel may be a specific capillary which can be placed into a capillary LightCycler instrument (Roche Applied Science cat. No. 023 531 414 001).

### Step b)

In the context of the present invention, the term "PCR reaction buffer" which is added at step b) of the present invention is understood as any liquid in which the sample later on can be subjected to a PCR reaction without any intermediate purification step. The said second volume of the PCR reaction buffer added should be at least twice (2x) as large and preferably 5 times (5x) as large as the first volume. This will allow for efficient amplification of the target nucleic acid during the subsequent PCR reaction.

In one embodiment, the "PCR reaction buffer" already contains all compounds which are required for the performance of a PCR reaction, which are a thermostable DNA polymerase, a mixture of desoxynucleoside-triphosphates (dNTPs), and at least one appropriate pair of amplification primers. The "PCR reaction buffer" may also comprise an appropriate pH buffering compound (e.g. Tris), a Mg2+ salt, a hot start component and the like.

In a specific embodiment, the "PCR reaction buffer" may already comprise compounds that are required for monitoring the amplification of the target DNA in real time. In particular, these compounds are either fluorescent hybridzation probes or a double stranded DNA binding dye. The specifics of various possible detection formats will be discussed below.

Alternatively, it is also within the scope of the invention, if some, any or all of the aforementioned components or any other additional compounds are added subsequently to the lysis step c) but prior to the performance of the actual amplification reaction according to step d).

### Step c)

According to the present invention, lysis of the cells takes place within the PCR reaction buffer, without any prior addition of a specific lysis step reagent conventionally used in the art. Rather, lysis of the one or more living cells takes place by means of incubating the sample for a period of at least 30 seconds at at least 90°C. Usually, a period of 30 seconds of high temparature incubation is sufficient for a complete lysis of a moderate number of cells (not more than 64 cells) which enables for subsequent amplification and detection of single copy genes. However, it is also within the scope of the present invention, if said period is prolonged up to a period of 30 but preferably not more than 15 minutes and most preferably not more that 3 minutes. Excellent results are under most conditions obtained, if said period is at least 1 minute.

The temperature used for cell lysis should not exceed 100°C and preferably be 95°C or less because at higher temperatures there is an increasing risk that the components contained in the reaction buffer which are required for the subsequent PCR reaction are destroyed. For example even thermostable DNA polymerases such as Taq DNA polymerase are becoming substantually denatured or degraded at temperatures above 100°C.

### Step d)

As evidenced by the examples, a PCR reaction can be performed using the lysate directly without intermediate purification step. Dependent on the embodiment, however it is within the scope of the present invention, if additional compounds required for said PCR reaction are added to the sample subsequent to the lysis.

Since the present invention is applicable for analysis nucleic acids originating from only very few or even single cells, it is advantageous for the design of an experiment according to the present invention, if consideration is given to the ratio between the number of cells analyzed and the volume in which the actual PCR reaction according to step d) takes place. On the one hand side, the PCR reaction should be performed in a minimal volume in order to achieve an optimal degree of sensitivity if such a low amount of starting material shall be analyzed. Thus, it has been proven to be advantageous, if the ratio of the number of said cells of step a) versus the liquid volume in which the polymerase chain reaction of step d) is perfomed is at least 1 cell/ 20µl PCR reaction volume.

On the other hand, since there is no intermediate purification step, the lysed sample will contain cellular debris which may interfere with the efficiency of the PCR reaction. In this context, it has been proven to be advantageous, if the ratio of the number of said living cells or cell equivalents of step a) versus the liquid volume in which the poymerase chain reaction of step d) is perfomed is not greater than 2 cells/µl. Even more advantageous is a ratio between 1 cell/25µl and 2 cells/µl. As it has been determined experimentally a ratio within this range anables single copy analysis on DNA originating from only 1 single cell as well as a much higher cell numbers.

The PCR reaction may be a conventional PCR reaction, wherein the set comprises the target DNA, dNTPs, DNA Polymerase, which is preferably a DNA dependent DNA polymerase, an appropriate pH buffering system such as Tris and some other accessory compounds such as Mg2+ salts and the like.

In a specific embodiment, the Polymerase may also be a thermostable DNA Polymerase which is also capable of performing 1-step RT PCR in order to use the inventive method for monitoring of gene expression.

Analysis of the amplified DNA is subsequently achieved usually by means of Gel Electrophoresis. However, in one embodiment, the PCR reaction may be a real time PCR reaction, wherein the progress of amplification is continously monitored using, for example any of the following detection formates:
- **TaqMan Hydrolysis probe format:**
   A single-stranded Hybridization Probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured. TaqMan probe assays are disclosed in detail in US 5,210,015, US 5,538,848, and US 5,487,972. TaqMan hybridization probes and compound mixtures are disclosed in US 5,804,375.
   In a specific embodiment, the Taqman hybridization probes are UPL probes from the Universal Probe Library as available from Roche Applied Sciences (Cat. 2010/2011, p. 577).
- **Molecular Beacons:**
   These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).
- - **FRET hybridization probes:**
   The FRET Hybridization Probe test format is especially useful for all kinds of homogenous hybridization assays (Matthews, J.A., and Kricka, L.J., Analytical Biochemistry 169 (1988) 1-25). It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively to monitoring the increase in fluorescence of the FRET acceptor component, it is also possible to monitor fluorescence decrease of the FRET donor component as a quantitative measurement of hybridization event.
   In particular, the FRET Hybridization Probe format may be used in real time PCR, in order to detect the amplified target DNA. Among all detection formats known in the art of real time PCR, the FRET-Hybridization Probe format has been proven to be highly sensitive, exact and reliable (WO 97/46707; WO 97/46712; WO 97/46714). As an alternative to the usage of two FRET hybridization probes, it is also possible to use a fluorescent-labeled primer and only one labeled oligonucleotide probe (Bernard, P.S., et al., Analytical Biochemistry 255 (1998) 101-107). In this regard, it may be chosen arbitrarily, whether the primer is labeled with the FRET donor or the FRET acceptor compound.
- **Double Strand DNA binding dye format:**
   It is also within the scope of the invention, if real time PCR is performed in the presence of an additive according to the invention in case the amplification product is detected using a double stranded nucleic acid binding moiety. For example, the respective amplification product can also be detected according to the invention by a fluorescent DNA binding dye which emits a corresponding fluorescence signal upon interaction with the double-stranded nucleic acid after excitation with light of a suitable wavelength. The dyes SybrGreenI and SybrGold (Molecular Probes) are frequently used in the art. Another particularly useful dye is the LightCycler 480 Resolight dye (Roche Applied Science Cat. No: 04 909 640 001).
- **Melting curve analysis:**
   Due to the fact that real time amplicon detection with SybrGreen format can not discriminate between specific products and amplification artefacts such as primer/dimers, a subsequent melting curve analysis is usually performed. After completion of the PCR-reaction, the temperature of the sample is constitutively increased, and fluorescence is detected as long as SybrGreen is bound to the double stranded DNA present in the samples. Upon dissociation of the double stranded DNA the signal decreases immediately. This decrease is monitored with an appropriate fluorescence versus temperature-time plot such that a first derivative value can be determined, at which the maximum of fluorescence decrease is observed. Since primer/dimer double stranded DNAs are usually short, dissociation into single stranded DNA occurs at lower temperatures as compared to the dissociation of the double stranded specific amplification product.
   Moreover, also Molecular Beacons and FRET hybridization probes are used for melting curve analysis. After completion of the PCR-reaction, the temperature of the sample is constitutively increased, and fluorescence is detected as long as the hybridization probes are bound to the target DNA. At melting temperature, the hybridization probes are released from their target, and the fluorescent signal is decreasing immediately down to the background level. This decrease is monitored with an appropriate fluorescence versus temperature-time plot such that a first derivative value can be determined, at which the maximum of fluorescence decrease is observed.

### Hot start PCR

The PCR reaction set up may contain some compounds which are providing a hot start effect, i.e. the inhibition of unspecific primer anneling and subsequent elongation at ambient temperature, which occasionally results in unspecific amplification products such as primer dimer formation. Upon temperature increase, this inhibition becomes eliminated due to release of the hot start compound from any binding partner with the consequence that the thermostable DNA Polymerase is becoming thermally activiated and specific polymerase catalyzed primer extension can occur. Many examples for such compounds are known in the art. A specific example is given in US 5,338,671, which discloses a Taq Polymerase antibody as a hot start compound. More recent examples for such hot start compounds are disclosed in EP 1 989 324 A and EP 2 163 556.

Said hot start compounds may be added to the sample subsequent to lysis together with the polymerase and any other PCR compounds prior to step d). Preferably, however, said hot start compounds are included already within the "PCR reaction buffer" that is being added during step b). As a consequence the termal acitivation of the thermostable DNA polymerase can already be achieved through the incubation at at least 90°C during step c).
In a particular embodiment, the DNA polymerase is reversibly inactivated as a result of a chemical modification. More precisely, heat labile blocking groups are introduced into the Taq DNA polymerase which renders the enzyme inactive at room temperature (US 5,773,258). These blocking groups are removed at high temperature during a pre-PCR step such that the enzyme is becoming activated. Such a heat labile modification, for example can be obtained by coupling Citraconic Anhydride or Aconitric Anhydride to the Lysine residues of the enzyme (US 5,677,152). Enzymes carrying such modifications are meanwhile commercially available as Amplitaq Gold (Moretti, T., et al., Biotechniques 25 (1998) 716-22) or FastStart DNA polymerase (Roche Applied Sciences Cat No: 12 032 902 001). Addition of FastStart DNA polymerase as part of the "PCR reaction buffer" and subsequent activation during lysis step c) has been proven to be a particularily efficient embodiment of the present invention.

### Kits

The present disclosure also includes a new typ of kits for performing real time PCR analysis. The kits comprise a reagent component and a disposable component, which together can be used and are specifically adapted for any of the methods disclosed above. At least, such a kit comprises
- a plurality of reaction vessels designed to fit into a thermocycler instrument,
- a thermostableDNA dependent DNA polymerase, and
- d-NTPs,
In addition, such a kit may comprise at least one pair of amplification primers,
The reaction vessels may be physically connected with each orther in a form of a microtiter plate or a linear strip of reaction vessels.
Also preferably, said thermosable Polymerase is thermally activiated by means of incubtion for at least 1 minute at 90°C. In a particular embodiment, the DNA polymerase is reversibly inactivated as a result of a chemical modification. More precisely, heat labile blocking groups are introduced into the Taq DNA polymerase which renders the enzyme inactive at room temperature (US 5,773,258). These blocking groups are removed at high temperature during a pre-PCR step such that the enzyme is becoming activated. Such a heat labile modification, for example can be obtained by coupling Citraconic Anhydride or Aconitric Anhydride to the Lysine residues of the enzyme (US 5,677,152). Enzymes carrying such modifications are meanwhile commercially available as Amplitaq Gold (Moretti, T., et al., Biotechniques 25 (1998) 716-22) or FastStart DNA polymerase (Roche Applied Sciences Cat No: 12 032 902 001).

In a specific aspect of said kit said pair of amplification primers is designed to amplify a single copy gene from Genomic DNA. Thus,as a consequence, such a kit for the firs time provides to the scientist a useful tool containing a complete set of all reagents and disposables necessary for single copy gene analysis.

### Examples

### Example 1

### qPCR for amplification of the GAPDH gene and the RPLI13A gene from sorted mouse hybridoma cells

A defined number of Mouse Hybridoma cells was deposited into separate wells of a 96 well microtiter plate using a Cell Sorter (Beckton Dickinson, FACS Aria I) in such a way that the liquid beam was always oriented into the center of the well. Due to the underlying technology of the Cell Sorter, however, it could not be excluded that a minor percentage of the sorted particles were not intact whole cells but cellular debris. Thus, in the following, the number of sorted material will be termed cell equivalent.

Cells sorted as disclosed were distributed into a 96 well microtiter plate (Roche Applied Science Cat. No: 04 729 692 001) designed for the LC480 real time PCR instrument (Roche Applied Science Cat. No: 05 015 278 001) according to the following pipetting scheme:
1 cell equivalent/well of column 1-4
2 cell equivalents/well in column 5-6
4 cell equivalents/well in column 7-8
8 cell equivalents/well in column 9
16 cell equivalents/well in column 10
32 cell equivalents/well in column 11
64 cell equivalents/well in column 12

To each well, a master mix was added which contained
0.4 µM Forward primer agcttgtcatcaacgggaag (SEQ ID NO: 1)
0.4 µM Reverse primer tttgatgttagtggggtctcg (SEQ ID NO: 2)
0.2 µM UPL Probe (RocheApplied Science Cat. No: 04 685 075 001, No. 9)
1x LC480 Probe Master (Roche Applied Science Cat. No: 04 902 343 001)

The forward and reverse primer were designed to amplify the mouse GAPDH gene, which is known to be present in the mouse genome in high copy numbers.

On a separate plate the same master mix was added, but primers and probe were designed to amplify the gene RPLI13A, which is present in only 12 copies of the mouse genome. Primers and probe were as follows:
0.4 µM Forward primer catgaggtcgggtggaagta (SEQ ID NO: 3)
0.4 µM Reverse primer gcctgtttccgtaacctcaa (SEQ ID NO: 4)
0.2 µM UPL Probe (RocheApplied Science Cat. No: 04 686 993 001, No.25)

The LightCycler Probe Master comprises the thermostable FastStart DNA polymerase, which is a hot start enzyme that is chemically modified. Activation is induced by means of removing said modification through incubation at high temperature.

qPCR was performed in an LC480 real time PCR instrument according to the following thermocycling protocol:

| | | | |
|---|---|---|---|
| Preincubation: | 1x | 95°C | 10' |
| Denaturation | 45x | 95°C | 10" |
| Annealing | 45x | 60°C | 30" |
| Elongation | 45x | 72°C | 1" |
| Cooling ramp rates | 2.2°C/s | | |
| Heating ramp rates | 4.4°C/s | | |

Detection of amplification signals and calculation of cp values (low cp values indicating a high level of amplification) was performed according to the instructions of the Manufacturer's manuals.

The following table discloses the average cp values obtained for the different cell numbers analyzed:

| Number of cells | Average cp Value | |
|---|---|---|
| | GAPDH | RPLI13A |
| 1 | 33,00 | 36.10 |
| 2 | 31.40 | 34,00 |
| 4 | 31.28 | 33,92 |
| 8 | 29,38 | 33,35 |
| 16 | 29,02 | 31,67 |
| 32 | 28,46 | 31,37 |
| 64 | 28,29 | 30,99 |

As can be seen from the table, signals originating from the high copy number mouse GAPDH gene as well as the RPLI13A gene, which is present in 12 copies in the mouse genome, can be detected even if only 1 cell is used as a starting material.

Moreover, it can be observed that the cp values inversely correlate with the number of cells/per well. Thus, it can be obviously concluded that addition of the PCR reaction buffer and subsequent incubation for 10' at 95°C was obviously enough to lyse the cells in a quantitative manner.

### Example 2

### qPCR for amplification of the Kcnj2 gene from sorted mouse hybridoma cells

The experiment was essentially carried out as disclosed for example 1 with the alteration that primers and probe were designed to amplify the single copy mouse gene Kcnj2. Primers and probe were as follows:
Forward primer ctgtcttgccttcgtgctct (SEQ ID NO: 5)
Reverse primer agcagggctatcaaccaaaa (SEQ ID NO: 6)
UPL Probe (RocheApplied Science Cat. No: 04 688 996 001, No.76)

The table discloses the average cp values obtained for the different cell numbers analyzed:

| Number of cells | Average cp value |
|---|---|
| 1 | 38,31 |
| 2 | 36,64 |
| 4 | 36,26 |
| 8 | 36,10 |
| 16 | 33,93 |
| 32 | 32,88 |
| 64 | 34,03 |

As can be seen from the table, amplification signals originating from the single copy number mouse gene Kcnj2 can be obtained, even if only one cell is used as a starting material. In other words, the present invention provides a solution for amplification of single copy genes from single cell samples.

Moreover, it can be observed that the cp value increases, if the sample originates from a higher cell number such as 64 cells. This can be explained by the fact that due to the lysis within the PCR reaction buffer at 95°C, the concentration of cell debris within the given reaction volume increases and thus may inhibit amplification efficiency of the PCR reaction. It can be concluded that the present inventions is especially applicable for PCR on samples originating from lower cell numbers.

Furthermore, the following table discloses the cp values obtained from individual single cell samples:

| | | | | | | | |
|---|---|---|---|---|---|---|---|
| 40,00 | 36,96 | 36,71 | 35,92 | - | 40,00 | - | - |
| 37,71 | 40,00 | - | 40,00 | 39,56 | 37,19 | 37,22 | 38,50 |

As it can be deduced from the table, no amplification signals were obtained in about 4 out of 16 parallel reactions. Taking the results of example 2 into account, which proves that not every single sorting event results in the actual separation and delivery of a single cell into a reaction vessel this result is explainable. In other words, the fact that in some cases no amplificationsignal is observed is due to the fact that the individual wells did not contain a cell, but it is not due to the fact that the lysis and amplification procedure itself has a certain failure rate.

### Example 3

### qPCR for amplification of the Kcnj2 gene from sorted mouse hybridoma cells using microtiter plates containing dried reagents

The experiment was essentially performed as disclosed for example 2 with the following alterations:
10 µl of a solution containing the required primers and probe was filled into each well of a microtiter plate. The microtiter plate was incubated for 12h at 25°C and 200 mBar, and subsequently for 4 h at 25°C and 50 mBar, so that the primers and probes were dried onto the surface of each reaction well of the microtiter plate.

Subsequently, cell deposition was performed as follows:
1 cell equivalent/well of column 1-6
2 cell equivalents/well in column 7
4 cell equivalents/well in column 8
8 cell equivalents/well in column 9
16 cell equivalents/well in column 10
32 cell equivalents/well in column 11
64 cell equivalents/well in column 12

After addition of 20µl master mix, the real time PCR analysis was performed. The table discloses the average cp values obtained for the different cell numbers analyzed:

| Number of cells | Average cp value |
|---|---|
| 1 | 38,14 |
| 2 | 37,45 |
| 4 | 36,22 |
| 8 | 35,14 |
| 16 | 34,89 |
| 32 | 33,76 |
| 64 | 33,27 |

It is also important to note that from the 48 wells used for single cell analysis, only 10 amplification reactions were negative. These reactions were not included into the calculation of the average cp value.

### Example 4

### qPCR for amplification of the Kcnj2 gene from single mouse hybridoma cells using microtiter plates containing dried reagents

In order to analyze, how much percentage of cell equivalent actually corresponds to a living cell rather then to cellular debris, 3 x 30 sorted equivalents were deposited each on a microscopic slide and counted. 28, 28 and 29 cells, respectively could be identified by visual inspection through a microscope. This corresponds to 94% living cells versus 6 % cellular debris per cell equivalent (sorting event).

In the following the experiment was essentially performed on two microtiter plates as disclosed for example 3 with the alteration, that on both microtiter plates, each reaction well only contained a single cell equivalent. Results were as follows:

| Plate No. | Number of wells without detectable amplification | Percentage of wells with detectable amplification | Average cp value of wells with detectable amplification | Standard deviation cp value |
|---|---|---|---|---|
| 1 | 16/96 | 84% | 38,85 | 1,14 |
| 2 | 5/96 | 95% | 38,31 | 1,01 |

The results show that it is possible to perform single cell analysis according to the PCR method as provided by the present invention. Moreover, also if single cell analyis is intended, the primers and probe are dried onto the surface of the microtiter plate.

## Claims

1. Method for amplification of a single copy DNA target nucleic acid from one single living eucaryotic cell of human, animal or plant origin, comprising the steps of
a) transfering a liquid sample with a first volume comprising the single living eucaryotic cell into a a well of a microtiter plate,
b) adding to said well a PCR reaction buffer with a second volume, whereas said second volume is at least 2x as large as said first volume and the final volume does not exceed 25 µl, said buffer comprising at least one pair of amplification primers, a thermostable DNA polymerase and dNTPs,
c) incubating said well for at least 30 seconds to not more than 15 minutes at at least 90°C, thereby lysing the living cell in the PCR reaction buffer without any prior addition of a specific lysis step reagent and activating the activity of said thermostable DNA polymerase,
d) amplifying said target by means of a polymerase chain reaction with a thermostable DNA dependent DNA polymerase without performance of an intermediate purification step,
wherein steps a) to d) are performed within the same reaction vessel.

2. Method according to claim 1, **characterized in that** said thermostable DNA Polymerase carries a chemical modification which is removed from said polymerase during step c).

3. Method according to claims 1-2, **characterized in that** prior to step a) said liquid comprising the single living cell has been gained by a cell sorting method.

## Patentansprüche

1. Verfahren zur Amplifikation einer Einzelkopie-DNA-Ziel-Nukleinsäure aus einer einzelnen lebenden eukaryotischen Zelle menschlichen, tierischen oder pflanzlichen Ursprungs, umfassend die folgenden Schritte
a) Überführen einer flüssigen Probe mit einem ersten Volumen, umfassend die einzelne lebende eukaryotische Zelle, in eine Vertiefung einer Mikrotiterplatte,
b) Zugeben eines PCR-Reaktionspuffers mit einem zweiten Volumen in die Vertiefung, wobei das zweite Volumen mindestens 2x so groß ist wie das erste Volumen und das Endvolumen 25 µl nicht übersteigt, wobei der Puffer mindestens ein Paar von Amplifikationsprimern, eine thermostabile DNA-Polymerase und dNTPs umfasst,
c) Inkubieren der Vertiefung für mindestens 30 Sekunden bis nicht mehr als 15 Minuten bei mindestens 90 °C, wodurch die lebende Zelle in dem PCR-Reaktionspuffer ohne jegliche vorherige Zugabe eines speziellen Lyseschrittreagens lysiert und die Aktivität der thermostabilen DNA-Polymerase aktiviert wird,
d) Amplifizieren des Ziels mittels einer Polymerase-Kettenreaktion mit einer von thermostabiler DNA abhängigen DNA-Polymerase ohne Durchführung eines Zwischenreinigungsschrittes,
wobei Schritte a) bis d) in demselben Reaktionsgefäß durchgeführt werden.

2. Verfahren nach Anspruch 1, **dadurch gekennzeichnet, dass** die thermostabile DNA-Polymerase eine chemische Modifikation trägt, die während Schritt c) aus der Polymerase entfernt wird.

3. Verfahren nach den Ansprüchen 1-2, **dadurch gekennzeichnet, dass** vor Schritt a) die Flüssigkeit, die die einzelne lebende Zelle umfasst, durch ein Zellsortierungsverfahren erhalten wurde.

## Revendications

1. Méthode d'amplification d'une copie unique d'un acide nucléique cible de type ADN à partir d'une cellule eucaryote vivante unique d'origine humaine, animale ou végétale, comprenant les étapes de
a) transfert d'un échantillon liquide ayant un premier volume comprenant la cellule eucaryote vivante unique dans un puits d'une plaque de microtitration,
b) ajout audit puits d'un tampon de réaction de PCR ayant un second volume, considérant que ledit second volume est au moins 2x plus important que ledit premier volume et que le volume final ne dépasse pas 25 µl, ledit tampon comprenant au moins une paire d'amorces d'amplification, une ADN polymérase thermostable et des dNTP,
c) incubation dudit puits pendant au moins 30 secondes jusqu'à pas plus de 15 minutes à au moins 90 °C, ce qui permet de lyser la cellule vivante dans le tampon de réaction de PCR sans ajout préalable d'un réactif d'étape de lyse spécifique et activation de l'activité de ladite ADN polymérase thermostable,
d) amplification de ladite cible par le biais d'une réaction en chaîne par polymérase avec une ADN polymérase dépendant d'un ADN thermostable sans mise en œuvre d'une étape de purification intermédiaire,
dans laquelle les étapes a) à d) sont mises en œuvre dans le même récipient réactionnel.

2. Méthode selon la revendication 1, **caractérisée en ce que** ladite ADN polymérase thermostable porte une modification chimique qui est éliminée de ladite polymérase pendant l'étape c).

3. Méthode selon les revendications 1 à 2, **caractérisée en ce qu'**avant l'étape a) ledit liquide comprenant la cellule vivante unique a été obtenu par une méthode de tri cellulaire.


### SEQUENCE LISTING

<110> Roche Diagnostics GmbH
   F. Hoffmann-La Roche AG
<120> Method for cell lysis in a PCR reaction buffer
<130> 27059 EP-HIL
<150> EP10186416
   <151> 2010-10-04
<160> 4
<170> PatentIn version 3.5
<210> 1
   <211> 20
   <212> DNA
   <213> Artificial Sequence
<220>
   <223> forward primer
<400> 1
   agcttgtcat caacgggaag 20
<210> 2
   <211> 21
   <212> DNA
   <213> Artificial Sequence
<220>
   <223> reverse primer
<400> 2
   tttgatgtta gtggggtctc g 21
<210> 3
   <211> 20
   <212> DNA
   <213> Artificial Sequence
<220>
   <223> forward primer
<400> 3
   catgaggtcg ggtggaagta 20
<210> 4
   <211> 20
   <212> DNA
   <213> Artificial Sequence
<220>
   <223> reverse primer
<400> 4
   gcctgtttcc gtaacctcaa 20